# EUROPEAN PATENT SPECIFICATION

(11) **EP 1 660 041 B1**
(45) Date of publication and mention of the grant of the patent: **06.03.2013**
(21) Application number: 04776989.8
(22) Date of filing: 23.06.2004
(51) Int. Cl.: A61K 9/127, A61K 41/00, A61P 35/00

(54) **NON-POLAR PHOTOSENSITIZER FORMULATIONS FOR PHOTODYNAMIC THERAPY**
NICHTPOLARE PHOTOSENSIBILISATOR-FORMULIERUNGEN FÜR DIE PHOTODYNAMISCHE THERAPIE
FORMULATIONS D'AGENT PHOTOSENSIBILISANT NON POLAIRE POUR LA THERAPIE PHOTODYNAMIQUE

(30) Priority: 26.08.2003 US 648168
(43) Date of publication of application: 31.05.2006
(60) Divisional application: 12161556.1
(73) Proprietor: Biolitec Pharma Marketing Ltd, F.T. Labuan (MY)
(72) Inventor: ALBRECHT, Volker, 07749 Jena (DE); FAHR, Alfred, 35091 Cölbe/Marburg (DE); SCHEGLMANN, Dietrich, 07751 Heba (DE); GRÄFE, Susanna, 07745 Jena (DE); NEUBERGER, Wolfgang, 87000 F.T. Labuan (MY)
(74) Representative: Horner, Martin Grenville
(86) International application number: PCT/US2004/020163
(87) International publication number: WO 2005/023220

(56) References cited:
- WO-A2-01/08660
- GB-A- 2 146 525
- US-A- 5 389 378
- US-A- 5 389 378
- US-A- 6 074 666
- US-A- 6 074 666
- US-A1- 2002 061 330
- DOBLER-GIRDZIUNAITE D ET AL: "kombinierte Anwendung der photodynamischen Therapie mit ionisierenden Strahlen bei Mamma-karzinomzellen in vitro [The combined use of photodynamic therapy with ionizing radiation on breast carcinoma cells in vitro]" STRAHLENTHERAPIE UND ONKOLOGIE, URBAN UND VOGEL, MUENCHEN, DE, vol. 171, no. 11, 1 November 1995 (1995-11-01), pages 622-629, XP008116046 ISSN: 0179-7158

## Description

### Background of the Invention

### 1. Field of the Invention

The present invention concerns the preparation of liposomal formulations containing Temoporfin or other non-polar photosensitizers and their use in therapy, particularly using intravenous injection.

### 2. Information Disclosure Statement

Liposomes are artificial vesicles composed of concentric lipid bilayers separated by water-compartments and have been extensively investigated as drug delivery vehicles. Due to their structure, chemical composition and colloidal size, all of which can be well controlled by preparation methods, liposomes exhibit several properties which may be useful in various applications. The most important properties are colloidal size, i.e. rather uniform particle size distributions in the range from 20 nm to 10 µm, and special membrane and surface characteristics.

Liposomes are used as carriers for drugs and antigens because they can serve several different purposes (Storm & Crommelin, Pharmaceutical Science & Technology Today, 1, 19-31 1998). Liposome encapsulated drugs are inaccessible to metabolizing enzymes. Conversely, body components (such as erythrocytes or tissues at the injection site) are not directly exposed to the full dose of the drug. The Duration of drug action can be prolonged by liposomes because of a slower release of the drug in the body. Liposomes possessing a direction potential, that means, targeting options change the distribution of the drug over the body. Cells use endocytosis or phagocytosis mechanism to take up liposomes into the cytosol. Furthermore liposomes can protect a drug against degradation (e.g. metabolic degradation). Although sometimes successful, liposomes have limitations. Liposomes not only deliver drugs to diseased tissue, but also rapidly enter the liver, spleen, kidneys and Reticuloendothelial Systems, and leak drugs while in circulation (Harris & Chess, Nature, March 2003, 2, 214-221).

Pegylation is an alternative method to overcome these deficiencies. First, pegylation maintains drug levels within the therapeutic window for longer time periods and provides the drug as a long-circulating moiety that gradually degrades into smaller, more active, and/or easier to clear fragments. Second, it enables long-circulating drug-containing micro particulates or large macromolecules to slowly accumulate in pathological sites with affected vasculature or receptor expression and improves or enhances drug delivery in those areas. Third, it can help to achieve a better targeting effect for those targeted drugs and drug carriers which are supposed to reach pathological areas with diminished blood flow or with a low concentration of a target antigen. The benefits of pegylation typically result in an increased stability (temperature, pH, solvent, etc.), a significantly reduced immunogenicity and antigenicity, a resistance to proteases, a maintenance of catalytic activity, and improvements in solubility, among other features, and an increased liquid stability of the product and reduced agitation-induced aggregation.

Liposome membranes containing bilayers-compatible species such as poly (ethylene glycol)-linked lipids (PEG-lipid) or gangliosides are being used to prepare stealth liposomes (Papahadjopoulos et al., PNAS, 88, 11460-4 1991). Stealth liposomes have a relatively long half-life in blood circulation and show an altered biodistribution in vivo. Vaage et al. (Int. J. of Cancer 51, 942-8, 1992) prepared stealth liposomes of doxorubicin and used them to treat recently implanted and well established growing primary mouse carcinomas, and to inhibit the development of spontaneous metastases from intra-mammary tumor implants. They concluded that long circulation time of the stealth liposomes of doxorubicin formulation accounts for its superior therapeutic effectiveness. The presence of MPEG-derivatized (pegylated) lipids in the bilayers membrane of sterically stabilized liposomes effectively furnishes a steric barrier against interactions with plasma proteins and cell surface receptors that are responsible for the rapid intravascular destabilization/rupture and RES clearance seen after i.v. administration of conventional liposomes. As a result, pegylated liposomes have a prolonged circulation half-life, and the pharmacokinetics of any encapsulated agent are altered to conform to those of the liposomal carrier rather than those of the entrapped drug (Stewart et al., J. Clin. Oncol. 16, 683-691, 1998). Because the mechanism of tumor localization of pegylated liposomes is by means of extravasation through leaky blood vessels in the tumor (Northfelt et al., J. Clin. Oncol. 16, 2445-2451, 1998; Muggia et al., J. Clin. Oncol. 15, 987-993, 1997), prolonged circulation is likely to favor accumulation in the tumor by increasing the total number of passes made by the pegylated liposomes through the tumor vasculature.

Photodynamic therapy (PDT) is one of the most promising new techniques being explored for use in a variety of medical applications and is known as a well-recognized treatment for the destruction of tumors ("Pharmaceutical development and medical applications of porphyrin-type macrocycles", T.D. Mody, J. Porphyrins Phthalocyanines, 4, 362-367 2000). Another important application of PDT is the treatment of infectious diseases due to pathogenic micro organisms including dermal, dental, suppurative, respiratory, gastro enteric, genital and other infections.

A constant problem in the treatment of infectious disease is the lack of specificity of the agents used for the treatment of disease, which results in the patient gaining a new set of maladies from the therapy.

The use of PDT for the treatment of various types of disease is limited due to the inherent features of photosensitizers. These include their high cost, extended retention in the host organism, substantial skin photo toxicity, background toxicity, low solubility in physiological solutions (which reduces its usefulness for intravascular administration as it can provoke thromboembolic accidents), and low targeting effectiveness. These disadvantages lead to the administration of extremely high doses of a photosensitizer, which dramatically increase the possibility of accumulation of the photosensitizer in non-damaged tissues and the accompanying risk of affecting non-damaged sites.

One of the prospective approaches to increase the specificity of photosensitizers and the effectiveness of PDT is a conjugation of a photosensitizer with a ligand-vector, which specifically binds to receptors on the surface of a target cell. A number of natural and synthetic molecules recognized by target cells can be used as such vectors. This approach is now used in the design of new generations of photosensitizers for the treatment of tumors ("Porphyrin-based photosensitizers for use in photodynamic therapy" E.D. Sternberg, D. Dolphin, C. Brueckner, Tetrahedron, 54, 4151-4202 1998).

Another approach to increase tumor selectivity by targeting photosensitizers to tumor cells is using liposomes, e.g. transferrin-conjugated liposomes (Derycke & De Witte, Int. J. Oncology 20, 181-187, 2002). Because non-conjugated liposomes are often easily recognized and eliminated by the reticuloendothelial system, PEG-ylated liposomes were used (Woodle & Lasic, Sterically stabilized liposomes, Biochim Biophys Acta 1113, 171-199, 1992; Dass et al., Enhanced anticancer therapy mediated by specialized liposomes. J Pharm Pharmacol 49, 972-975, 1997).

Since the application of photodynamic therapy in the treatment of cancer and other diseases is increasing rapidly, there is also a bigger demand for new photosensitizer formulations. These new photosensitizer formulations need to be more stable, easy to manufacture and to handle. Furthermore, especially more hydrophobic non-polar photosensitizers, should be able to target tissue in an efficient and selective manner.

US6,074,666 discloses porphyrin photosensitizer liposome formulations, which comprise one or more phospholipids together with a disaccharide or polysaccharide. Monosaccharides are not preferred.

### Objectives and Brief Summary of the Invention

It is an objection of embodiments of the present invention to provide an improved photosensitizer formulation for use in photodynamic therapy (PDT).

It is another object to incorporate non-polar photosensitizers into a liposomal membrane thus allowing non-polar and polar substances to be transported using the same vehicle.

It is a further object to preserve the structure and size of liposomal constructs with incorporated non-polar photosensitizer during freeze-drying processes by adding monosaccharides or polyalcohols as cryoprotectants.

It is yet another object to provide a photosensitizer formulation with improved pharmacokinetic properties.

It is still another object to improve the transport of non-polar photosensitizers through the cell membrane and thus increasing the efficacy of PDT.

The present invention provides a freeze-dried pharmaceutical liposomal formulation for photodynamic therapy comprising a liposomal bilayer, which consists substantially of phospholipids selected from the group consisting of dipalmitoyl phosphatidyl choline, dipalmitoyl phosphatidyl glycerol and combinations of these materials; a polyalcohol or a monosaccharide selected from the group consisting of glucose and fructose; and a therapeutically effective amount of a non-polar photosensitizer selected from dihydro- and tetrahydro-porphyrins.

The present invention involves a pharmaceutical liposomal formulation for photodynamic therapy comprising a non-polar porphyrin photosensitizer and one or more phospholipids, which are stable in storage without requiring freeze-drying. The liposomal formulation provides therapeutically effective amounts of the photosensitizer for intravenous administration. The formed liposomes contain the non-polar photosensitizer within the membrane and are useful for the combined targeting of a non-polar photosensitizer and a second polar substance. When a formulation includes the presence of monosaccharides or polyalcohols, it can be efficiently freeze-dried preserving the size of the liposomal vehicles and the content of a therapeutically effective amount of the photosensitizing agent. The invention also relates to the liposome composition formed upon reconstitution with an aqueous vehicle. The freeze-dried formulation upon reconstitution with a suitable aqueous vehicle forms liposomes that are also useful for intravenous administration.

Preferred embodiments will now be described by way of example.

### Brief Description of Figures

Figure 1 is a gel filtration curve of liposomal formulated mTHPC. Both, lipid components and mTHPC show the same distribution over all fractions collected.
Figure 2 displays the light-induced fluorescence (LIF) measurement of Colo 26 tumors with intact skin, Swiss nu/nu mice 0.5 mg/kg, with intravenous injection of mTHPC, product A and B giving the accumulation in tumor as a function of time post-injection.
Figure 3 shows the PDT-effect 6 h after intravenous injection of product A, B and mTHPC. Mice were injected intraperitoneally (i.p.). (0.25 ml) with Evans blue (1%) immediately after PDT treatment and sacrificed after 24 h.

### Detailed Description of Preferred Embodiments

A pharmaceutical liposomal formulation for photodynamic therapy comprising a non-polar porphyrin photosensitizer and one or more phospholipids, which are stable in storage is described below. The liposomal formulation provides therapeutically effective amounts of the photosensitizer for intravenous administration. The formed liposomes contain the non-polar photosensitizer within the membrane and are useful for the combined targeting of a non-polar photosensitizer and a second polar substance. Also disclosed is such formulation which is stable in 8 forage without requirring freeze-drying.

The formulation is preserved by a freeze-drying procedure using a monosacchande or polyalcohol, but need not be to have a reasonably stable shelf life. The process cycle preserves the size of the liposomal vehicle as well as the membrane content of the photosensitizing compound.

The non-polar photosensitizers in this invention include the known porphyrin based compounds. In particular photosensitizers, that are advantageously employed in the practice of this invention, are based upon porphyrin macrocycle photosensitizers, which include deuteroporphyrin, etioporphyrin, protoporphyrin, hematoporphyrin, pheophorbide and derivatives thereof, especially di- and tetra-hydroporphyrins, that have light absorption maxima in the range of 640-780 nanometers.

The formed liposomes contain the non-polar photosensitizer within the membrane and are useful for the combined targeting of a non-polar photosensitizer and a second polar substance.

The photosensitizing formulations are useful to target the non-polar photosensitizer molecule to the unwanted cells or tissues or other undesirable materials and, after irradiation with an appropriated light source, to damage the target. The photosensitizing formulations are also useful to monitor unwanted cells or tissues or other undesirable materials by using fluorescence imaging methods without or with limited photochemical activation of the photosensitizer.

Especially the liposomal formulation of the invention is useful to transport non-polar photosensitizers. Non-polar substances are integrated within the membrane of the vehicles, thereby creating a structure that opens up easier, freeing the photosensitizer for action directly to the cell membrane. This mechanism delivers the photosensitizer directly to the cellular membrane system, one preferred place of action. Thus the photosensitizer, being effectively activated by illumination with an appropriate external light source, can irreversibly damage unwanted cells, tissues or other structures.

Conventionally constructed liposomal formulations are used to transport several compounds trapped into the luminal part of the vehicle. The invention focuses on the combination of two different transport compartments within one liposome allowing a combined transport of non-polar and polar substances. In this way, by residing photosensitizer within the membrane, photosensitizing agents are effectively targeted to their place of action but the luminal part inside the liposome particle stays free for the inclusion of other substances, including drugs that may have a beneficial effect on the therapy.

In addition, the combination of monosaccharides as glucose and the photosensitizer attached to phospholipids is an excellent tool to preserve the size of the liposomes during freeze-drying and rehydration using a physiological common carbohydrate instead of disaccharides.

This versatile concept allows the addition of substances, that will have a beneficial effect on the therapy. The combination of two or more substances together with a membrane bound photosensitizer within one vehicle will allow one to directly or indirectly influence the oxygen content of the targeted cells thereby influencing the efficacy of PhotoDynamic therapy. For instance, such effects can be achieved by adding inhibitors of enzyme activities like inhibitors of the mammalian thioredoxin reductase (TrxR). Thus, effective only after reaching the cytoplasm of the targeted cells, the inhibitor will block the Trx/TrxR pathway which normally acts as an antioxidant.

### Example 1

### Preparation of Liposomes containing m-THPC

mTHPC (Temoporfin) was synthesized as described in Pat. No. 4,992,257 and 5,162,519.

Disclosed are liposomes prepared according to the following general procedure:

Non-polar photosensitizer, ascorbic palmitate and the phospholipids are dissolved in chloroform/methanol. The solution is then dried under vacuum using a rotary evaporator until the chloroform/methanol mixture is not detectable by gas chromatography anymore. Water for injection is added to rehydrate the lipid film at a temperature of 50°C for at least 2 hours. The mixture is then passed through a homogenizer filter system using a final pore size of 100 nanometer. Optionally, the rehydration water is supplemented with monosaccharides or polyalcohols. The filtrate is collected, filled into vials and optionally freeze dried. The freeze dried composition is reconstituted with water for injection prior to administration.

Using the foregoing procedure, several different preparations of the liposomal formulation were prepared as follows:

### Example 1a (Reference)

| Ingredient | Amount % w/v |
|---|---|
| mTHPC | 0.05 to 0.15 |
| Dipalmitoyl Phosphatidyl Choline | 0.5 to 2.0 |
| Dipalmitoyl Phosphatidyl Glycerol | 0.05 to 0.2 |
| pegylated Distearoyl Phosphatidyl Ethanolamine | 0.05 to 0.2 |
| Ascorbic Palmitate | 0.002 to 0.004 |
| Water for Injection | as required to achieve desired concentrations above |

### Example 1b (Reference)

| Ingredient | Amount % w/v |
|---|---|
| mTHPC | 0.05 to 0.15 |
| Dipalmitoyl Phosphatidyl Choline | 0.5 to 2.0 |
| Dipalmitoyl Phosphatidyl Glycerol | 0.05 to 0.2 |
| Ascorbic Palmitate | 0.002 to 0.004 |
| Water for Injection | as required to achieve desired concentrations above |

### Example 1c

| Ingredient | Amount % w/v |
|---|---|
| mTHPC | 0.05 to 0.15 |
| Dipalmitoyl Phosphatidyl Choline | 0.5 to 2.0 |
| Dipalmitoyl Phosphatidyl Glycerol | 0.05 to 0.2 |
| Glucose | 2.0 to 12.0 |
| Ascorbic Palmitate | 0.002 to 0.004 |
| Water for Injection | as required to achieve desired concentrations above |

All were found to function well in their use according to the present invention.

### Example 2

### Physical and Chemical Stability of Liposomal m-THPC

The physical stability of the liposomal formulations was measured by monitoring the particle size distribution by photon correlations spectroscopy.

### Stability of liposomal mTHPC

| Storage Conditions | Mean Particle Size distribution (nm) |
|---|---|
| Initial | 166 |
| 23°C - 1 Month | 177 |
| 23°C - 4 Month | 167 |

### Example 3

### Localization of mTHPC within the liposomal bilayer of the formulation

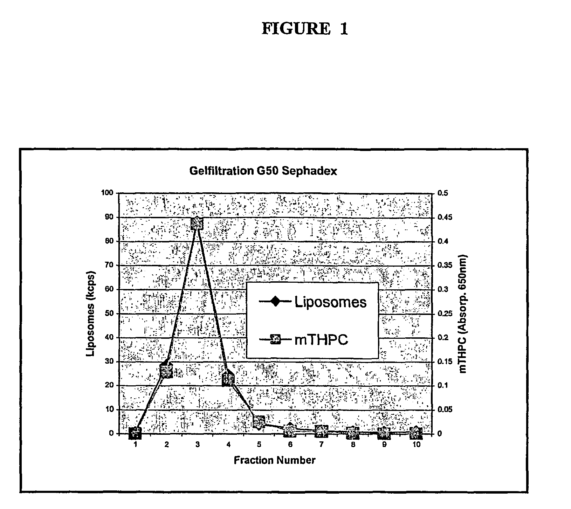

Gel filtration of liposomal formulation performed on Sephadex G50 columns. As shown in Figure 1, lipids and mTHPC show the same distribution over all fractions indicating a physically interaction of both components i.e. integration of mTHPC into the membrane bilayer. The distribution does not change markedly after destroying the liposomal structure by ultrasonication, as shown in Tables 1 and 2.

### Example 4

### Pharmacokinetic properties in mice

Colo 26, a non-metastasizing mouse colorectal tumor cell line, syngeneic to Balb/c mice, was used.

Cells were maintained as a monolayer culture in Roswell Park Memorial Institute (RPMI)-1640 medium completed with 10% heat-inactivated fetal calf serum, 1% streptomycin-penicillin and 200 mML-glutamine at 37° in 95% air and 5% CO₂. Six-week athymic female mice (Swiss, nu/nu) were inoculated subcutaneously into the right hind foot with 2 x 10⁶ Colo26 cells. Two weeks later, as the tumor reached a diameter of 5-7 mm, formulations of m-THPC (0.5 mg/kg) were injected intravenous.

Non-invasive LIF measurements have been performed at three distinct sites: tumor with overlaying skin, symmetric normal tissues with overlaying skin on the left hind foot and elevated skin at different times after drug administration. At the selected time points mice were sacrificed and the fluorescence signals from tumor and normal tissues were measured in direct contact.

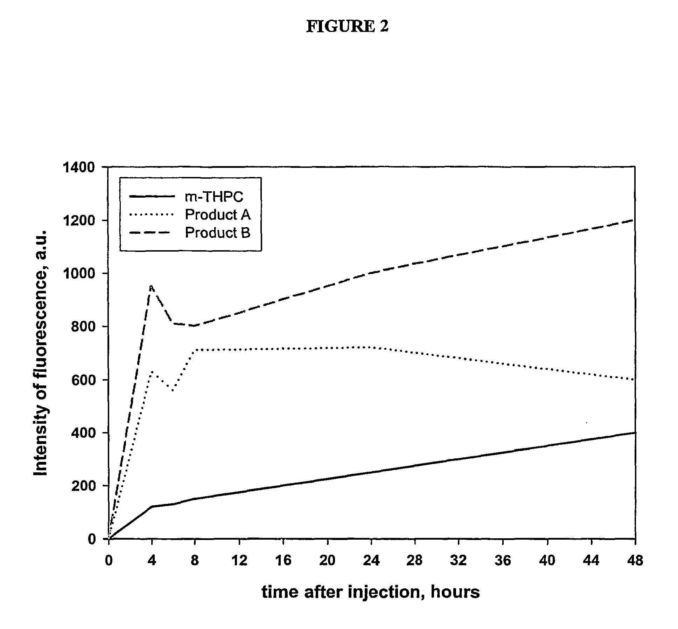

Figure 2 displays the accumulation of mTHPC, product A and B in tumor as a function of time post-injection measured in four mice. Non-invasive measurements demonstrated the trend for the better accumulation of mTHPC in the tumor compared to normal tissues with a best ratio (1.3) at 24h. Invasive measurements performed in the direct contact with the tumor and muscle at 24h and 48h post-injection gave the ratios of 2.7 and 3.0 respectively.

Measurements for product A have been done in one mouse at 0.5h, 4h, 6h, 8h, 12h, 24h, 36h, 48h, and 72h post-dose. Product A has shown similar pharmacokinetic behavior like mTHPC. Compared to mTHPC the highest fluorescence intensity in tumor tissue was already reached after 4 hours after injection.

Measurements for Product B have been done in one mouse at 0.5h, 4h, 6h, 8h, 12h, 24h, 36h, 48h, and 72 post-dose. The highest fluorescence intensity in tumor was measured 8 hours after i.v. injection. In comparison to mTHPC product B is showing completely different pharmacokinetic properties. The concentration of Product B was increased in tumor much faster (16h) than mTHPC.

### Example 5

### Antitumor Activity of Liposomal m-THPC

Colo 26, a non-metastasizing mouse colorectal tumor cell line, syngeneic to Balb/c mice, was used.

Cells were maintained as a monolayer culture in Roswell Park Memorial Institute (RPMI)-1640 medium completed with 10% heat-inactivated fetal calf serum, 1% streptomycine-penicillin and 200 mM L-glutamine at 37° in 95% air and 5% CO₂.

Six-week athymic female mice (Swiss, nu/nu) were inoculated subcutaneously into the right hind foot with 2 x 10⁶ Colo26 cells. Two to three weeks later, as the tumour reached a diameter of 10-13 mm, formulations of m-THPC (0.5 mg/kg) were injected intravenous. Unless otherwise indicated, 3 mice per group per product were used for each post-PDT.

### a. Photodynamic Treatment

Drug-light intervals (DLI) of 0.5h, 4h, 6h, 72h were used for the products as mTHPC, product A (liposomes and m-THPC) and product B (PEG-ylated liposomes and m-THPC). Each animal was anaesthetized by i.m. injection of ketamine (12.5mg/ml), then photoirradiated at 652 nm with 10 J/cm² at 100 mW/cm² using a light diode laser.

### b. Evaluation of PDT effect

### Morphological method

To evaluate the tumor tissue necrosis after PDT treatment, Evans Blue dye method was used.

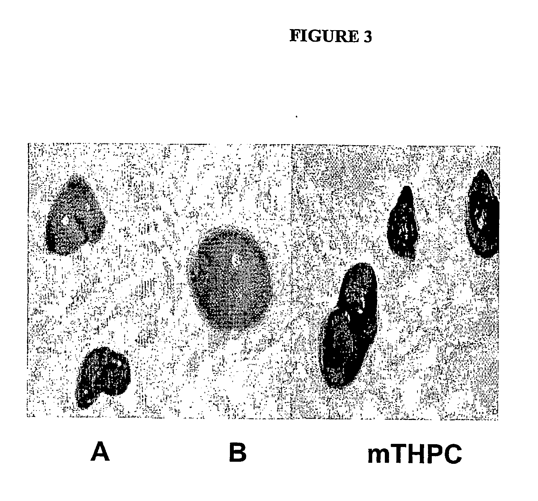

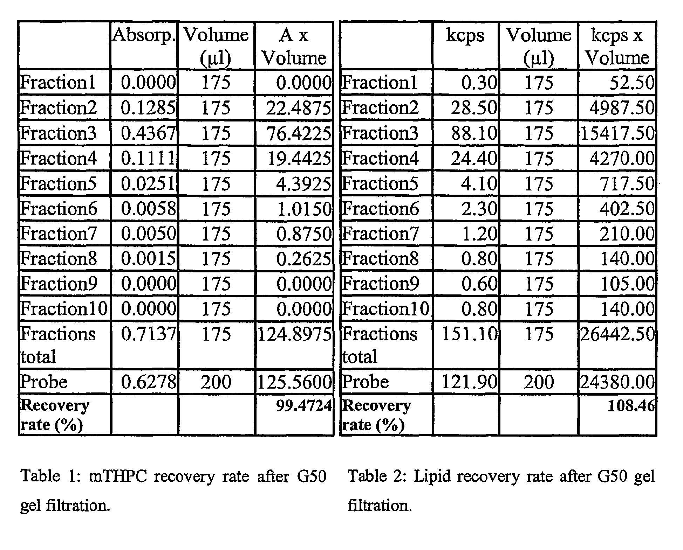

Mice were injected i.p. (0.25 ml) with Evans blue (1%) immediately after PDT treatment.
Twenty four hours later the mice were sacrificed with an overdose of halothan, tumors were ablated and cut longitudinally. As exemplified in Figure 3, the photos of the whole tumors and tumor slices had been taken.

Unstained necrotic areas were considered as tumor damage:
i. Drug-light interval (DLI) = 0.5h, necrosis was not observed irrespective to the products used.
ii. Drug-light interval (DLI) = 4h. Three mice treated with mTHPC-PDT demonstrated partial tumor necrosis. Two mice treated with product A gave partial tumor necrosis, while the tumor of the third mouse remained intact. Three mice treated with product B-PDT demonstrated tumor necrosis: in two mice the distinct tumor necrosis was observed, while in the one mouse the tumor necrosis was partial.
iii. Drug-light interval (DLI) = 6h; Six mice were used for the product A. Distinct necrosis was noticed in the tumors of two mice treated with product A and four others were intact. For the product B, a distinct tumor necrosis was observed in all mice. Two tumors treated with mTHPC-PDT demonstrated distinct necrosis, one tumor was free of necrosis. mTHPC-PDT tumors that displayed necrosis were confirmed by ocular observations.

## Claims

1. A freeze-dried pharmaceutical liposomal formulation for photodynamic therapy comprising a liposomal bilayer, which consists substantially of phospholipids selected from the group consisting of dipalmitoyl phosphatidyl choline, dipalmitoyl phosphatidyl glycerol and combinations of these materials; a polyalcohol or a monosaccharide selected from the group consisting of glucose and fructose; and a therapeutically effective amount of a non-polar photosensitizer selected from dihydro- and tetrahydro-porphyrins.

2. A liposomal formulation according to claim 1, which consists of dipalmitoyl phosphatidyl choline and dipalmitoyl phosphatidyl glycerol in amounts of 0.5 - 2.0 and 0.05 - 0.2 % w/v, respectively, and amounts of temoperfin, glucose, and ascorbic palmitate in amounts of 0.05 - 0.15, 2.0-12.0, and 0.002 - 0.004 % w/v, respectively and water for injection as required to achieve desired concentrations.

3. A liposomal formulation according to claim 1, wherein the non-polar photosensitizer is Temoporfin.

4. A liposomal formulation according to claim 1, wherein the weight ratio of phospholipid to monosaccharide is between 1:2 and 1:12.

5. A liposomal formulation according to claim 1 wherein the therapeutically effective concentration of the photosensitizer is from 0.0001 to 0.15 percent w/v.

6. A liposomal formulation according to claim 1, reconstituted with an aqueous fluid for pharmaceutical administration.

7. A liposomal formulation according to claim 1 further comprising a component selected from the group consisting of butylated hydroxytoluene, ascorbic palmitate, and combinations of these two.

## Patentansprüche

1. Eine gefriergetrocknete pharmazeutische liposomale Formulierung für die photodynamische Therapie beinhaltend: eine liposomale Doppelschicht, die im Wesentlichen aus Phospholipiden besteht, wobei diese ausgewählt sind aus einer Gruppe bestehend aus Dipalmitoylphosphatidylcholin, Dipalmitoylphosphatidylglycerin und Kombinationen dieser Materialien; einem Polyalkohol oder einem Monosaccharid ausgewählt aus einer Gruppe bestehend aus Glucose und Fructose; und einer therapeutisch wirksamen Menge eines unpolaren Photosensibilisators ausgewählt aus einer Gruppe bestehend Dihydro- und Tetrahydroporphyrinen.

2. Eine liposomale Formulierung gemäß Anspruch 1, bestehend aus Dipalmitoylphosphatidylcholin und Dipalmitoylphosphatidylglycerin in Anteilen von 0.5 - 2,0 bzw. 0,05 - 0,2 % w/v, und aus Temoporfin, Glucose, und Ascorbinsäurepalmitat in Anteilen von 0,05 - 0,15, 2,0 - 12,0, bzw. 0,002-0,004% w/v, und aus Wasser für Injektionszwecke in entsprechender Menge, um die gewünschten Konzentrationen zu erreichen.

3. Eine liposomale Formulierung gemäß Anspruch 1, wobei der unpolare Photosensibilisator Temoporfin ist.

4. Eine liposomale Formulierung gemäß Anspruch 1, wobei das Gewichtsverhältnis zwischen Phospholipid und Monosaccharid zwischen 1:2 und 1:12 liegt.

5. Eine liposomale Formulierung gemäß Anspruch 1, wobei die therapeutisch wirksame Konzentration an Photosensibilisator zwischen 0,0001 und 0.15% w/v liegt.

6. Eine liposomale Formulierung gemäß Anspruch 1, rekonstituiert mit einer wässrigen Flüssigkeit für eine pharmazeutische Verabreichung.

7. Eine liposomale Formulierung gemäß Anspruch 1, umfassend darüber hinaus eine Komponente ausgewählt aus seiner Gruppe bestehend aus butyliertem Hydroxytoluen, Ascorbinsäurepalmitat, und Kombinationen dieser beiden.

## Revendications

1. Une formulation pharmaceutique liposomale lyophilisée destinée à être utilisée en thérapie photodynamique, comprenant une double couche liposomale, qui est constituée essentiellement :
- de phospholipides sélectionnés à partir d'un groupe constitué de dipalmitoyl phosphatidyl choline, de dipalmitoyl phosphatidyl glycérol ainsi que des combinaisons de ces matériaux ;
- d'un polyalcool ou d'une monosaccharide sélectionné à partir d'un groupe constitué de glucose et de fructose;
- ainsi que d'une quantité thérapeutiquement efficace d'un photosensibilisant non polaire sélectionné à partir de dihydro- et tetrahydro-porphyrines.

2. Une formulation liposomale selon la revendication 1, qui est constituée de dipalmitoyl phosphatidyl choline et de dipalmitoyl phosphatidyl glycérol dans une quantité de 0,5 - 2,0 et de 0,05 - 0,2 % p/v, respectivement, et d'une quantité de témoporfine, de glucose, et de palmitate ascorbique en quantité de 0,05 - 0,15, 2,0 - 12,0, et 0,002-0,004% p/v, respectivement ainsi que de l'eau pour une injection selon les besoins afin d'obtenir la concentration souhaitée.

3. Une formulation liposomale selon la revendication 1, dans laquelle le phototosensibilisant non polaire est la Témoporfine.

4. Une formulation liposomale selon la revendication 1, dans laquelle le rapport entre le poids des phospholipides et des monosaccharides est compris entre 1:2 et 1:12.

5. Une formulation liposomale selon la revendication 1, dans laquelle la concentration thérapeutiquement efficace du photosensibilisant est de 0,0001 à 0,15 pour cent p/v.

6. Une formulation liposomale selon la revendication 1, reconstituée avec un liquide aqueux pour une administration pharmaceutique.

7. Une formulation liposomale selon la revendication 1, comprenant en outre un composant choisi dans le groupe constitué par de l'hydroxytoluène butylé, du palmitate ascorbique et des combinaisons de ces deux composants.
